(19)

Europäisches Patentamt
European Patent Office
Office européen des brevets

(11) **EP 4 083 820 A1**

(12) **EUROPEAN PATENT APPLICATION**

(43) Date of publication:
**02.11.2022 Bulletin 2022/44**

(21) Application number: **21179162.9**

(22) Date of filing: **13.06.2021**

(51) International Patent Classification (IPC):
**G06F 17/16** (2006.01)   **G01N 33/00** (2006.01)
**G16B 40/00** (2019.01)

(52) Cooperative Patent Classification (CPC):
**G06F 17/16; G06K 9/6247;** G01N 21/359;
G01N 30/02; G01N 2021/3595; G01N 2201/129;
G16B 40/10

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **30.04.2021   IT 202100011123**

(71) Applicant: **Analytical Solutions S.r.l.**
**00198 Roma (IT)**

(72) Inventors:
• **Risoluti, Roberta**
  **00198 Roma (IT)**
• **Materazzi, Stefano**
  **00198 Roma (IT)**

(74) Representative: **Leganza, Alessandro**
**Via Sottopasso, 14**
**36040 Torri di Quartesolo (VI) (IT)**

(54)   **SYSTEM AND METHOD TO IDENTIFY AND QUANTIFY INDIVIDUAL COMPONENTS IN A SAMPLE BEING ANALYZED**

(57)   The present invention is located in the field of systems and methods for processing analytical data capable of identifying and/or quantifying individual components, individual analytes or other components which may be present in the samples, present in a plurality of samples to be analyzed.

Fig. 1

EP 4 083 820 A1

**Description**

**Technical field**

The present invention is located in the field of systems and methods for processing analytical data capable of identifying and/or quantifying individual components, individual analytes or other components which may be present in the samples, present in a plurality of samples to be analyzed.

**State of the art**

The qualitative and quantitative analysis of samples to be analyzed has attracted the interest of the scientific community for centuries. Determining the composition of a sample of matter in qualitative terms, i.e., determining the individual constituent compounds and/or determining the relative amount thereof within the sample has always been a difficult problem to solve, especially in the case of complex matrices. In particular, for example, revealing the presence and corresponding relative quantity of selected components (such as small molecules, therapeutic agents, xenobiotics, metabolites and other substances) has been an objective of researchers and physicians for a long time.

To this end, studies have been carried out for decades in order to develop increasingly sophisticated analytical instruments with lower and lower compound detection limits.

If the sample subjected to spectrometry contains substantially pure components (for example, metabolites of small molecules), the spectrum of the component may be easily matched to the spectra of known components in order to identify the component. Furthermore, if a single ion for a specific component is present, the intensity (as distinguishable in the spectrum) of the ion may be used for the relative quantification of the component in the sample. However, in many cases, fractionation of a particular sample, for example by means of a liquid or gas chromatograph, is often incomplete. For example, two or more compounds of small similar molecules may "co-elute" in the process of chromatographic separation, resulting in an impure mixture of components which enters in the spectrometer, for example in the mass spectrometer. Therefore, subtle spectral trends observed on many samples of the same type may be indicative of the presence of one or more otherwise obscured components.

Furthermore, certain analytical methods were proposed to quantitatively analyze the spectrometer data sets in a group of samples. For example, factor analysis (FA), principal component analysis (PCA) and singular value decomposition (SVD) have been applied to a matrix of spectrometer data from a group of samples, for example biological samples, to generate a small number of basic spectral profiles (corresponding to single compounds in the samples) and to calculate the weights with which each of these basic components is present in a single sample. However, the analytical methods FA, PCA and SVD provide results which are often ambiguous and/or difficult to interpret, for the basic spectral profiles may include several negative values (without any significant analytical value). Therefore, post-analysis transformations, which require further computational power, time and skill, are needed to collect physically significant analytical results from the process. Furthermore, the analytical methods FA, PCA and SVD do not necessarily provide results which aim at independent groups of components present in the samples, indicative of particular metabolic compounds or other components present in the samples, as described for example by Juvela et al. See Juvela, M., Lehtinen, K. e Paatero, P., "The Use of Positive Matrix Factorization in the Analysis of Molecular Line Spectra from the Thumbprint Nebula (1994), "Clouds Cores and Low Mass Starts ASP Conference Series, Vol. 65, pp. 176-180; D. P. Clemens e R. Barvainis, eds.

Therefore, there is the need for an improved system and method for solving the technical issues outlined above which are associated with systems for analyzing existing analytical data. More particularly, there is the need for a system and method capable of analyzing the data provided to the analytical instrumentation of a group of samples to easily and accurately determine: physically relevant quantities of each individual component, often a compound, for example a metabolite present in the samples, regardless of the coelution of some similar compounds in a particular sample. It is further required a system and a method for processing analytical instrumental data to determine the relative concentration of individual constituents or individual compounds in the samples.

**Summary of the invention**

The system and method of the present invention allows for the qualitative and quantitative analysis of analytes in complex matrices, based on chemometric data processing algorithms, allowing the development of new analytical platforms for the characterization and typification of products intended for a specific high-level market. The primary objective is to bring innovation in safety and quality control of a product, for example in the food and no-food sector, through a model which is easy to use as it is unique and non-specific, capable of being used on any data matrix and therefore capable of processing instrumental signals obtained by means of analytical instrumentations and therefore by means of analyses with different analytical techniques.

In detail, the analytical protocols of the invention involve analyzing the samples by recording instrumental signals with different technologies and then processing the data through chemometric calculus algorithms aimed at developing qualitative and quantitative prediction models. The method of the invention allows to provide simple, quick and fast analytical protocols wherein, by selecting a suitable range of variables and a data pretreatment algorithm, the user of the method is capable of carrying out accurate analyses through a "one-touch" technology. Such technology will allow to obtain qualitative and quantitative responses by comparing the instrumental signal of the sample with that of a reference calibration set, by using a unique method suitable for all instrumental signals.

There are numerous papers in the literature reporting applications of mathematical algorithms that use chemometrics to develop specific prediction models according to the matrix being analyzed and the instrumental signal.

Object of the present invention is a system and a method titled "Universal Non-linear Iterative Correlation" (abbreviated as UNICO), capable of being universally used for processing different instrumental signals while appropriately choosing two variables in a defined range.

With respect to existing literature, the method of the invention may be used for all instrumental signals and does not require optimization of specific data treatment protocols for each signal and thus for each analytical technique. Thus, the advantage of the present invention in the field of quality assurance of food and no-food products lies precisely in the possibility for the user of the method to carry out accurate and fast analyses without having to modify the method according to the analytical application, as well as ensuring the standardization of data processing procedures in order to reduce the measurement error.

The problem addressed by the present invention is thus that of providing a system and a method which would allow to identify and/or quantify individual components in samples being analyzed without having to modify the method according to the analytical application, effectively solving all the situations listed above.

Therefore, the present invention solves the above-mentioned problem by means of the system and the method employed as outlined in the appended claims, the definitions of which are an integral part of the present description, thus allowing to have a system and a method which further allow to identify and/or quantify individual components in samples being analyzed.

Further features and advantages of the system of the invention and of the methods of use will result from the description of the examples of embodiments of the invention, provided as an indication of the invention.

**Brief description of the Figures**

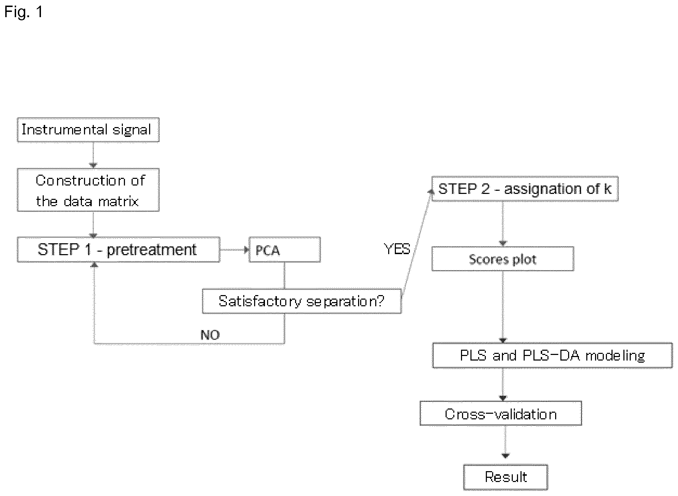

Fig. 1 exemplifies the method of the invention. The representation of the method is carried out by means of a block diagram.

**Detailed description of the invention**

For the purposes of the description herein, the term "and/or", if used in a list of two or more articles, means that any one of the listed articles may be employed alone, or in any combination of two or more of the listed articles.

For example, if a combination is described as containing the components A, B and/or C, or A and/or B and/or C, the composition may contain only A; only B; only C; a combination of A and B; a combination of A and C; a combination of B and C; or a combination of A, B and C.

The terms "comprises", "comprising" or any other variation thereof, intend to cover a non-exclusive inclusion, such that a system, a method, a use, etc. which comprises a list of elements does not only include those elements, but may include other elements not explicitly listed or pertaining to such system, method, use, etc.

An element followed by "comprises...a..." does not preclude, without further limitations, the existence of further identical elements in the system, method or use which comprises the element.

An object of the present invention is a system to identify and/or quantify individual components in samples being analyzed comprising or, alternatively, consisting of:

    a) an analytical instrument;

    b) a data acquisition module configured to receive data relating to the chemical analysis of a sample being analyzed by the analytical instrument a);

    c) a data processing module, in communication with the data acquisition module b), configured for:

        - organizing the data of module b) in a matrix where the columns represent the observed functions and the rows are the considered variables,

        - performing the principal component analysis through the NIPALS algorithm according to the following equation 1:

$$X = TP^T + E \qquad \text{eq. 1}$$

where X is the data matrix (m x n) to be analyzed, T is the score matrix (m x a), P the load matrix (n x a) and E the residual.

- performing the data pretreatment through one of the following mathematical algorithms:

  ○ column scaling,
  ○ column centering,
  ○ column standardization,
  ○ column autoscaling,
  ○ standard normal variable;

- optionally, if the separation between the samples is not satisfactory, the previous step is repeated using a combination of the above-mentioned mathematical algorithms;
- selecting the variables in the data matrix including a number of points per sample not less than 2500 and not more than 4000 and selecting N-kR points, where N represents the number of variables, R represents the resolution factor and k is the correlation coefficient, which is comprised from 1 to 5, where k is determined in such a way as to minimize the distance between samples belonging to the same class.
- by running PLS regression algorithms on the data set of the original matrix, returning the identification and/or quantification of individual components in samples being analyzed;

d) a display and/or printing unit suitable for being connected to the processing module c) suitable for displaying and/or printing the obtained results.

The analytical instrument a) is selected from a spectrometer, mass spectrometer, nuclear magnetic resonance spectrometer, spectroscope, spectrophotometer, IR, FT-IR, NIR or micro-NIR spectrophotometer, thermogravimetry, chromatograph, gas chromatograph, liquid chromatograph.

In data acquisition form b) the data relating to the chemical analysis of a sample being analyzed may be:

- the absorbance as a function of wavelength, in case the analytical instrument a) is a spectrometer, spectroscope or a spectrophotometer,
- the mass variation expressed as a weight/weight percentage as a function of temperature, in case the analytical instrument a) is a thermogravimetry,
- the response intensity expressed as an area as a function of the retention time, in case the analytical instrument a) is a chromatograph.

The activities for which the module c) is configured may be carried out by means of a computer program.

The individual components in samples being analyzed are chemical compounds, small molecules, therapeutic agents, analytes, ions, chemical compounds in solid, liquid, solution or gas state, gene sequences, amino acid sequences or metabolites.

Another object is a method to identify and/or quantify individual components in samples being analyzed comprising or, alternatively, consisting of the following steps:

A. receiving in a data acquisition form b) the data relating to the chemical analysis of a sample being analyzed generated using an analytical instrument a),

B. in a processing module c), in communication with the data acquisition module b), organizing the data of step A. in a matrix where the columns represent the observed functions and the rows are the considered variables,

C. performing the principal component analysis through the NIPALS algorithm according to the following equation 1:

$$X = TP^T + E \qquad \text{eq 1}$$

where X is the data matrix (m x n) to be analyzed, T is the so-called score matrix (m x a), P the load matrix (n x a) and E the residual.

D. performing the data pretreatment through one of the following mathematical algorithms:

- column scaling,

- column centering,
- column standardization,
- column autoscaling,
- standard normal variable;

E. optionally, if the separation between the samples is not satisfactory, step D. is repeated using a combination of the above-mentioned mathematical algorithms;

F. selecting the variables in the data matrix including a number of points per sample of not less than 2500 and not more than 4000 and selecting N-kR points, where N represents the number of variables, R represents the resolution factor and k is the correlation coefficient, which is comprised from 1 to 5, where k is determined in such a way as to minimize the distance between samples belonging to the same class;

G. by performing PLS regression algorithms on the data set of the original matrix, returning the identification and/or quantification of individual components in samples being analyzed.

According to an embodiment, the method is carried out by means of the system described above, including possible preferred embodiments.

According to a preferred embodiment of the method, in step G. the data set is divided into two categories, the training set and the evaluation set by organizing the matrices of the independent variables X and the dependent ones Y and using the NIPALS algorithm for the decomposition of X and Y according to equation 2, assigning a numerical value of the observed variable or the value 0, 1 or -1 corresponding to the class to which the samples belong.

$$X = TP^T + E, \quad Y = UQ^T + F, \quad U = TB \qquad \text{eq. 2}$$

and solving equation 2 using the least squares method, using cross-validation methods for verifying the performances of the method and returning a number with the standard deviation or belonging to a specific class with relative measurement error.

The UNICO method involves constructing a data matrix by organizing the instrumental output signals, wherein the samples are allocated along the rows and in the columns the continuous variables are reported.

The system and/or the method of the present invention involves receiving instrumental signals, with particular reference to spectroscopic, thermogravimetric and chromatographic signals, in which the variable being studied is observed as a function of the variation of a characteristic parameter. With reference to the above-cited signals, absorbance as a function of wavelength, mass variation expressed in weight/weight percentage as a function of the temperature and intensity of response expressed as an area as a function of retention time will be recorded. Starting data are organized in a matrix where the columns represent the observations made and the rows are the variables considered for the phenomenon being analyzed.

The system and/or method includes organizing data in such format and then performing the principal component analysis using the NIPALS algorithm for reducing the dimensionality of the problem:

$$X = TP^T + E \qquad \text{eq. 1}$$

where X is the data matrix (m x n) to be analyzed, T is the so-called matrix of scores (m x a), P the matrix of variables (n x a) and E the matrix of residuals, wherein m is the vector of results, a is the number of principal components and n is the dimension of the original variables.

The system and/or method includes organizing data in such format and then performing the principal component analysis using the NIPALS algorithm for reducing the dimensionality of the problem:

For each sample of the data matrix, a step is performed using one of the algorithms reported below:

1) Column scaling

$$y_{iv} = \frac{x_{iv} - \min_v(x_{iv})}{\max_v(x_{iv}) - \min_v(x_{iv})}$$

Xiv - original value of the sample I for the variable v
Yiv - corresponding value obtained by transforming Xiv

2) Column centering

$$Y_{iv} = X_{iv} - \overline{X}_v$$

Xiv - original value of the sample I for the variable v
X - means of the original value of the variable v

3) Column standardization

$$Y_{iv} = X_{iv}/S_v$$

$X_{iv}$ - original value of the sample I for the variable v
$S_v$ - standard deviation of the original value of the variable v
$Y_{iv}$ - corresponding value obtained by transforming Xiv

4) Column autoscaling

$$Y_{iv} = \frac{X_{iv} - \overline{X}_v}{S_v}$$

$X_{iv}$ - original value of the sample I for the variable v
X - means of the original value of the variable v
$S_v$ - standard deviation of the original value of the variable v
$Y_{iv}$ - corresponding value obtained by transforming Xiv

5) SNV

$$Y_{iv} = \frac{X_{iv} - \overline{X}_i}{S_i}$$

$X_{iv}$ - original value of the sample I for the variable v
X - means of the original value of the variable for the sample i
$S_i$ - standard deviation of the original value of the variable i
$Y_{iv}$ - corresponding value obtained by transforming Xiv

If the separation between the samples is not satisfactory, optionally, the UN ICO method will repeat the above-mentioned step by using combinations of said mathematical pretreatments.

Then, the method will perform a further step by selecting a number of points according to the resolution of the signal and by iteratively assigning a correlation coefficient k comprised from 1 to 5. When the UNICO method manages to find the value of k which minimizes the distance between samples belonging to the same class, the system terminates the operation and proceeds with data processing by returning a graphic plot wherein each point represents the scores, that is the analyzed objects, in the space of the principal components, to which a value in terms of eigenvectors solutions of equation 1 will be assigned.

In this step the interactive selection of the variables in the data matrix occurs, including a number of points per sample not less than 2500 and not more than 4000 and selecting N-kR points, where N represents the number of variables, R represents the resolution factor and k is a correlation coefficient comprised from 1 to 5.

At this point, the UNICO method will perform the PLS regression algorithms on the data set of the original matrix, for the qualitative and quantitative analysis of the samples processed with the mentioned techniques. In this phase, the UNICO method divides the data set into two categories, the training set and the evaluation set by organizing the matrices of the independent variables X and the dependent ones Y and using the NIPALS algorithm for the decomposition of X and Y according to equation 2, assigning a numerical value of the observed variable or the value 0, 1 or -1 corresponding to the class to which the samples belong.

$$X = TP^T + E, \quad Y = UQ^T + F, \quad U = TB \qquad \text{eq. 2}$$

Equation 2 is solved by means of the least squares method, by using cross-validation methods for verifying the performances of the method according to the parameters reported in Tab 1 and 2 and will return a number with the standard deviation or belonging to a specific class with relative measurement error.

According to a preferred embodiment, the method is like that exemplified in the scheme of Fig. 1.

The UNICO method can work in self-learning mode, by implementing the reference data matrix and improving the measurement error in the prediction of unknown samples.

Another object is the use of an above-described system to identify and/or quantify individual components in samples being analyzed.

According to a preferred embodiment, the use of the above-described system is preferred in the food sector.

By the description reported above, the advantages of the system and/or method with respect to the state of the art are apparent: unique system and/or method wherein, by varying two parameters within an established range, the qualitative or quantitative analysis of analytes in complex matrices is performed, regardless of the instrumental signal and thus of the used technique and the type of matrix being analyzed.

EXPERIMENTAL SECTION

In order to verify the analytical performances and thus the abilities of the system and method of the present invention, the method and the system have been used for analyzing instrumental signals coming from different analytical instruments. The objective was that of using the method to identify the presence or absence of analytes in classification or quantification problems by comparing the results obtained through classic prediction models based on Partial Least Square Discriminant Analysis (PLS-DA) and Partial Least Square Regression (PLS) algorithms.

The results obtained through the system and method of the same data set, previously analyzed through different models which were optimized every time, showed a correlation expressed in terms of correlation coefficient of the predicted values (obtained with the system and method of the invention) with respect to the measured values (reference values from optimized models) not less than 0.9963 regardless of the instrumental signal and thus of the used analytical technique.

Example 1

By way of example, the results obtained using the system and method of the invention on two data sets relating to two different analytical issues and which required the use of different analytical techniques are reported. In the first case, the spectroscopic signal deriving from MicroNIR miniaturized instrumentation operating in the near infrared was recorded for hemp flour samples previously analyzed as calibration set for the instrumental calibration of a PLS quantitative prediction model of cannabinoids in food products deriving from the hemp agro-food chain (see literature reference: Risoluti, R., Gullifa, G., Battistini, A., Materazzi, S. Monitoring of cannabinoids in hemp flours by MicroNIR/Chemometrics, (2020) Talanta, 211, art. no. 120672).

Comparison of the performances of the models has been carried out considering the measurement error expressed as Root Mean Square Error (RMSE), the correlation coefficient $R^2$ and the detection limit expressed as Minimum Detectable Concentration (MDC). Using the system and method of the invention on the same data set allowed to obtain comparable analytical performances reported in Table 1 and a correlation of predicted values with respect to the measured values of 0.9912 on 10 real samples of commercial flours.

Table 1. Analytical performances obtained with the method of the literature (see above) and the method of the present invention.

| Parameter | Model | CBD | THC | CBG |
|---|---|---|---|---|
| RMSE | Literature ref. | 0.006 | 0.005 | 0.007 |
| | Present invention | 0,006 | 0.007 | 0.004 |
| $R^2$ | Literature ref. | 0.9741 | 0.9980 | 0.9873 |
| | Present invention | 0.9935 | 0.9941 | 0.9760 |
| MDC | Literature ref. | 0.001 | 0.001 | 0.001 |
| | Present invention | 0,001 | 0.001 | 0.001 |

Wherein CBD means cannabidiol, THC means tetrahydrocannabinol, CBG means cannabigerol.

Example 2

The second data set refers to applications of chemometric algorithms in the bioanalytical field for identifying hereditary red cell pathologies by recording instrumental signals such as thermograms (TGA) (see literature reference: Risoluti, R., Caprari, P., Gullifa, G., Sorrentino, F., Maffei, L., Massimi, S., Carcassi, E., Materazzi, S.; Differential diagnosis of hereditary hemolytic anemias in a single multiscreening test by TGA/chemometrics; (2020) Chemical Communications, 56 (55), pp. 7557-7560; and subsequent development of a PLS-DA classification model considering 4 classes of subjects for calibrating the instrumental response: healthy donors (H), pathological subjects suffering from sickle cell anemia (SCA), pathological subjects suffering from thalassemia (TAL), pathological subjects suffering from hereditary elliptocytosis (HE) and hereditary spherocytosis (HS). Comparison of the performances of the models (Table 2) has been carried out considering the measurement accuracy expressed as Non-Error Rate (NER %), the specificity (Sp) and the efficiency (Eff).

Table 2. Comparison of the analytical performances of the method of the present invention with the method of literature

| Parameter | Class | Ref [9] | Present invention |
| --- | --- | --- | --- |
| *NER* | CTR | 100.0 | 100.0 |
| | HS/HE | 80.0 | 96.6 |
| | TAL | 100.0 | 100.0 |
| | SCA | 100.0 | 100.0 |
| *Specificity* | CTR | 88.3 | 87.9 |
| | HS/HE | 100.0 | 100.0 |
| | TAL | 100.0 | 100.0 |
| | SCA | 100.0 | 100.0 |
| *Efficiency* | CTR | 96.8 | 93.5 |
| | HS/HE | 89.4 | 90.4 |
| | TAL | 100.0 | 100.0 |
| | SCA | 100.0 | 100.0 |

Wherein NER means Non-Error Rate (NER %), CTR means controls, HS/HE means hereditary spherocytosis/hereditary elliptocytosis, TAL means thalassemic subjects, SCA means sickle cell disease.

**Claims**

1. System to identify and/or quantify individual components in samples being analyzed comprising or, alternatively, consisting of:

   a) an analytical instrument;
   b) a data acquisition module configured to receive data relating to the chemical analysis of a sample being analyzed by the analytical instrument a);
   c) a data processing module, in communication with the data acquisition module b), configured for:

      - organizing the data of module b) in a matrix where the columns represent the observed functions and the rows are the considered variables,
      - performing the principal component analysis through the NIPALS algorithm according to the following equation 1:

$$X = TP^T + E \qquad \text{eq. 1}$$

where X is the data matrix (m x n) to be analyzed, T is the score matrix (m x a), P the load matrix (n x a) and E the residual.

- performing the data pretreatment through one of the following mathematical algorithms:

  ◦ column scaling,
  ◦ column centering,
  ◦ column standardization,
  ◦ column autoscaling,
  ◦ standard normal variable;

- optionally, if the separation between the samples is not satisfactory, the previous step is repeated using a combination of the above-mentioned mathematical algorithms;
- selecting the variables in the data matrix including a number of points per sample not less than 2500 and not more than 4000 and selecting N-kR points, where N represents the number of variables, R represents the resolution factor and k is the correlation coefficient, which is comprised from 1 to 5, where k is determined in such a way as to minimize the distance between samples belonging to the same class.
- by running PLS regression algorithms on the data set of the original matrix, returning the identification and/or quantification of individual components in samples being analyzed;

d) a display and/or printing unit suitable for being connected to the processing module c) suitable for displaying and/or printing the obtained results.

2. System according to claim 1, wherein the analytical instrument a) is selected from a spectrometer, mass spectrometer, nuclear magnetic resonance spectrometer, spectroscope, spectrophotometer, IR, FT-IR, NIR or micro-NIR spectrophotometer, thermogravimetry, chromatograph, gas chromatograph, liquid chromatograph.

3. System according to any of claims 1 to 2, in which in module b), the data relating to the chemical analysis of a sample being analyzed are:

   - the absorbance as a function of wavelength, in case the analytical instrument a) is a spectrometer, spectroscope or a spectrophotometer,
   - the mass variation expressed as a weight/weight percentage as a function of temperature, in case the analytical instrument a) is a thermogravimetry,
   - the response intensity expressed as an area as a function of the retention time, in case the analytical instrument a) is a chromatograph.

4. System according to any one of claims 1 to 3, in which the activities for which the module c) is configured are carried out by means of a computer program.

5. System according to any one of claims 1 to 4, in which the individual components in samples being analyzed are chemical compounds, small molecules, therapeutic agents, analytes, ions, chemical compounds in solid, liquid, solution or gas state, gene sequences, amino acid sequences or metabolites.

6. Method to identify and/or quantify individual components in samples being analyzed comprising or, alternatively, consisting of the following steps:

   A. receiving in a data acquisition form b) the data relating to the chemical analysis of a sample being analyzed generated using an analytical instrument a),
   B. in a processing module c), in communication with the data acquisition module b), organizing the data of step A. in a matrix where the columns represent the observed functions and the rows are the considered variables,
   C. performing the principal component analysis through the NIPALS algorithm according to the following equation 1:

$$X = TP^T + E \qquad \text{eq 1}$$

where X is the data matrix (m x n) to be analyzed, T is the so-called score matrix (m x a), P the load matrix (n x a) and E the residual.

D. performing the data pretreatment through one of the following mathematical algorithms:

- column scaling,
- column centering,
- column standardization,
- column autoscaling,
- standard normal variable;

E. optionally, if the separation between the samples is not satisfactory, step D. is repeated using a combination of the above-mentioned mathematical algorithms;

F. selecting the variables in the data matrix including a number of points per sample of not less than 2500 and not more than 4000 and selecting N-kR points, where N represents the number of variables, R represents the resolution factor and k is the correlation coefficient, which is comprised from 1 to 5, where k is determined in such a way as to minimize the distance between samples belonging to the same class;

G. by performing PLS regression algorithms on the data set of the original matrix, returning the identification and/or quantification of individual components in samples being analyzed.

7. Method according to claim 6, wherein said method is carried out by means of the system according to any one of claims 1 to 5.

8. Method according to any one of claims 6 to 7, wherein in step G. the data set is divided into two categories, the training set and the evaluation set by organizing the matrices of the independent variables X and the dependent ones Y and using the NIPALS algorithm for the decomposition of X and Y based on equation 2, assigning a numerical value of the observed variable or the value 0, 1 or -1 corresponding to the class to which the samples belong

$$X = TP^T + E, \quad Y = UQ^T + F, \quad U = TB \qquad \text{eq. 2}$$

and solving equation 2 using the least squares method, using cross-validation methods for verifying the performances of the method and returning a number with the standard deviation or belonging to a specific class with relative measurement error.

9. Use of a system according to any one of claims 1 to 5 to identify and/or quantify individual components in samples being analyzed.

10. Use according to the claim 9, in the food field.

Fig. 1

Europäisches
Patentamt

European
Patent Office

Office européen
des brevets

# EUROPEAN SEARCH REPORT

Application Number

EP 21 17 9162

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/67722 A1 (UMETRI AKTIEBOLAG [SE]; WOLD SVANTE [SE]) 29 December 1999 (1999-12-29) * page 1 - page 7; figures 4-6 * ----- | 1-10 | INV. G06F17/16 G01N33/00 G16B40/00 |
| X | WO 2018/111116 A2 (IDLETECHS AS [NO]) 21 June 2018 (2018-06-21) * paragraph [0117] - paragraph [0200]; figures 3-6 * ----- | 1-10 | |

TECHNICAL FIELDS
SEARCHED (IPC)

G06F
G01N
G16B

The present search report has been drawn up for all claims

| Place of search | Date of completion of the search | Examiner |
|---|---|---|
| Berlin | 26 November 2021 | Virnik, Elena |

EPO FORM 1503 03.82 (P04C01)

## ANNEX TO THE EUROPEAN SEARCH REPORT
## ON EUROPEAN PATENT APPLICATION NO.

EP 21 17 9162

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2021

| Patent document cited in search report | | Publication date | Patent family member(s) | | Publication date |
|---|---|---|---|---|---|
| WO 9967722 | A1 | 29-12-1999 | AU | 4812299 A | 10-01-2000 |
| | | | EP | 1090358 A1 | 11-04-2001 |
| | | | JP | 2002519763 A | 02-07-2002 |
| | | | US | 6754543 B1 | 22-06-2004 |
| | | | WO | 9967722 A1 | 29-12-1999 |
| WO 2018111116 | A2 | 21-06-2018 | US | 2020083902 A1 | 12-03-2020 |
| | | | WO | 2018111116 A2 | 21-06-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- The Use of Positive Matrix Factorization in the Analysis of Molecular Line Spectra from the Thumbprint Nebula. **JUVELA, M. ; LEHTINEN, K. E ; PAATERO, P.** Clouds Cores and Low Mass Starts ASP Conference Series. 1994, vol. 65, 176-180
- **RISOLUTI, R. ; GULLIFA, G. ; BATTISTINI, A. ; MATERAZZI, S.** *Monitoring of cannabinoids in hemp flours by MicroNIR/Chemometrics,* 2020

- **RISOLUTI, R. ; CAPRARI, P. ; GULLIFA, G. ; SORRENTINO, F. ; MAFFEI, L. ; MASSIMI, S. ; CARCASSI, E. ; MATERAZZI, S.** Differential diagnosis of hereditary hemolytic anemias in a single multiscreening test by TGA/chemometrics. *Chemical Communications,* 2020, vol. 56 (55), 7557-7560